# NEUE EUROPÄISCHE PATENTSCHRIFT

(11) **EP 1 276 451 B2**
(45) Veröffentlichungstag und Bekanntmachung des Hinweises auf die Entscheidung über den Einspruch: **04.06.2008**
(45) Hinweis auf die Patenterteilung: 08.06.2005
(21) Anmeldenummer: 01925556.1
(22) Anmeldetag: 18.04.2001
(51) Int. Cl.: A61K 8/60, A61K 8/42, A61K 8/37, A61Q 5/00, D06P 5/08

(54) **VERWENDUNG VON ZUCKERTENSIDEN UND FETTSÄUREPARTIALGLYCERIDEN**
USE OF SUGAR SURFACTANTS AND FATTY ACID PARTIAL GLYCERIDES
UTILISATION DE TENSIOACTIFS A BASE DE SUCRE ET DE GLYCERIDES PARTIELS D'ACIDES GRAS

(30) Priorität: 28.04.2000 DE 10020887
(43) Veröffentlichungstag der Anmeldung: 22.01.2003
(73) Patentinhaber: Henkel Kommanditgesellschaft auf Aktien, 40589 Düsseldorf (DE)
(72) Erfinder: KLEEN, Astrid, 40699 Erkrath (DE); HÖFFKES, Horst, 40595 Düsseldorf (DE); ROHLAND, Christa, 40468 Düsseldorf (DE); FRAUENDORF, Bianca, 42553 Velbert (DE)
(86) Internationale Anmeldenummer: PCT/EP2001/004375
(87) Internationale Veröffentlichungsnummer: WO 2001/097759

(56) Entgegenhaltungen:
- EP-A- 0 711 542
- EP-A- 0 750 899
- DE-A- 19 543 633
- DE-A- 19 619 645
- DE-A- 19 713 696
- DE-A- 19 805 703
- DE-A- 19 907 714
- DE-A- 19 914 926
- DE-A- 19 926 526
- US-A- 5 089 578
- US-A- 5 635 461
- US-A- 5 716 418
- ANON.: "Hair treatment agent with improved luster effect" RESEARCH DISCLOSURE, Nr. 40530, 10. Januar 1998 (1998-01-10), Seite 26 XP000772356
- Sichereitsdatenblatt gemäss 91/155/EWG - ISO 11014-1 für Lamesoft PO 65

## Beschreibung

Die Erfindung betrifft die Verwendung einer Wirkstoffkombination aus Zuckertensiden und Fettsäurepartialglyceriden zur Farbintensivierung und der Verbesserung der Waschechtheit von Färbungen keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen.

Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat nicht an Anstrengungen gefehlt, die Echtheit von Färbungen keratinischer Fasern zu verbessern. Eine Entwicklungsrichtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird.

Aus der EP 0 655 905 B1 ist die Verwendung von Alkylglycosiden in Färbemitteln bekannt. In der DE-OS 199 190 89 werden Haarfärbepräparate mit Zuckertensiden und Fettsäurepartialglyceriden beschrieben, welche die Haarstruktur kräftigen und dermatologisch gut verträglich sind. Es werden jedoch keinerlei Informationen zur Farbintensivierung und zur Waschechtheit offenbart.

Überraschenderweise wurde nun gefunden, daß durch den Einsatz der erfindungsgemäßen Wirkstoffkombination bestehend aus Zuckertensiden und Fettsäurepartialglyceriden die Waschechtheit und die Farbintensivierung von Färbungen insbesondere keratinischer Fasern signifikant gesteigert werden kann. Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird. Unter der Farbintensivierung im Sinne der Erfindung ist zu verstehen, daß die Färbung durch die Anwendung der erfindungsgemäßen Wirkstoffkombination deutlich an Farbstärke zunimmt.

Ein erster Gegenstand der vorliegenden Erfindung ist daher die Verwendung einer Wirkstoffkombination aus
a) mindestens einem Zuckertensid (A) ausgewählt aus der Gruppe, die gebildet wird von Alkyl- und Alkenyloligoglykosiden (A1) und Fettsäure-N-alkylpolyhydroxyalkylamiden (A2), und
b) mindestens einem Fettsäurepartialglycerid (B)
   zur Verbesserung der Waschechtheit von Färbungen von Fasern, insbesondere von keratinischen Fasern, sowie zur Steigerung der Farbintensität.

Die erfindungsgemäß verwendete Wirkstoffkombination verbessert die Waschechtheit von Färbungen auf künstlichen Fasern wie Polyestern und natürlichen Fasern wie Baumwolle und insbesondere keratinischen Fasern.

Unter keratinischen Fasern werden erfindungsgemäß Pelze, Wolle, Federn und insbesondere menschliche Haare verstanden.

Die erfindungsgemäße Wirkstoffkombination enthält als erste zwingende Komponente ein Zuckertensid (A).

Gemäß einer ersten Ausführungsform (A1) handelt es sich bei dem Zuckertensid um ein Alkyl- oder Alkenyloligoglykosid. Diese Zuckertenside stellen bekannte nichtionische Tenside gemäß Formel (I) dar,

R¹O-[G]ₚ (I)

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.
Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10.

Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Gemäß einer zweiten Ausführungsform (A2) der Erfindung handelt es sich bei dem Zukkertensid um ein Fettsäure-N-alkylpolyhydroxyalkylamid, ein nichtionisches Tensid der Formel (II), 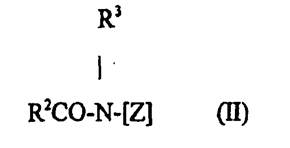 in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (III) wiedergegeben werden:

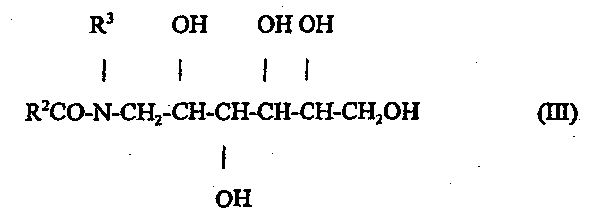

Vorzugsweise werden als Fettsäure-N-alkylpolyhydroxyalkylamide Glucamide der Formel (III) eingesetzt, in der R³ für Wasserstoff oder eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (III), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Das Zuckertensid ist in den erfindungsgemäß verwendeten Mitteln bevorzugt in Mengen von 0,1 - 20 Gew.-%, bezogen auf das gesamte Mittel, enthalten. Mengen von 0,5 - 5 Gew.-% sind besonders bevorzugt.

Die zweite zwingende Komponente der erfindungsgemäßen Wirkstoffkombination sind Fettsäurepartialglyceride. Diese Fettsäurepartialglyceride sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (IV), 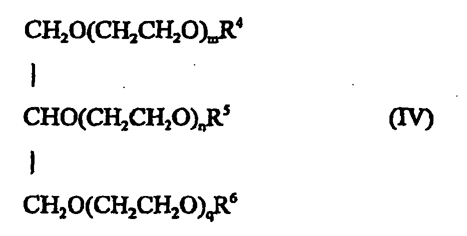 in der R⁴, R⁵ und R⁶ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R⁴ für einen Acylrest und R⁵ und R⁶ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure,

Gadoleinsäure, Behensäure und Erucasäure sowie deren technische Mischungen. Vorzugsweise werden Ölsäuremonoglyceride eingesetzt.

Das Fettsäurepartialglycerid ist in den erfindungsgemäß verwendeten Mitteln bevorzugt in Mengen von 0,1 - 20 Gew.-%, insbesondere 0,5 - 5 Gew.-%, bezogen auf das gesamte Mittel enthalten.

Die erfindungsgemäße Lehre umfaßt auch Ausführungsformen, in denen die Wirkstoffkombination mehrere Zuckertenside und/oder mehrere Fettsäurepartialglyceride enthält.

Als weiterhin vorteilhaft hat es sich gezeigt, daß Polymere die farberhaltende Wirkung des erfindungsgemäßen Wirkstoffkomplexes unterstützen können. In einer bevorzugten Ausführungsform werden den erfindungsgemäß verwendeten Mitteln daher Polymere zugesetzt, wobei sich kationische, anionische und amphotere Polymere als besonders wirksam erwiesen haben.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.
Homopolymere der allgemeinen Formel (V), 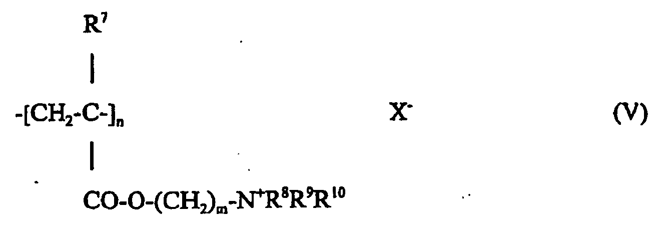 in der R⁷ = -H oder -CH₃ ist, R⁸, R⁹ und R¹⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (V) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R⁷ steht für eine Methylgruppe
- R⁸, R⁹ und R¹⁰ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (V) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquatemium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bei den anionischen Polymeren, welche die farberhaltende Wirkung der erfindungsgemäßen Wirkstoffkombination unterstützen können, handelt es sich um ein anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vemetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vemetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze® QM im Handel erhältlich.

Weiterhin können als Polymere zur Steigerung der Wirkung der erfindungsgemäßen Wirkstoffkombination amphotere Polymere verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (VI),

   R¹¹-CH=CR¹²-CO-Z-(CₙH₂ₙ)-N⁽⁺⁾R¹³R¹⁴R¹⁵ A⁽⁻⁾ (VI)

   in der R¹¹ und R¹² unabhängig voneinander stehen für Wasserstoff oder eine Methylgruppe und R¹³, R¹⁴ und R¹⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel (VII),

   R¹⁶-CH=CR¹⁷-COOH (VII)

   in denen R¹⁶ und R¹⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R¹³, R¹⁴ und R¹⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Es ist erfindungsgemäß auch möglich, daß die verwendeten Zubereitungen mehrere, insbesondere zwei verschiedene Polymere gleicher Ladung und/oder jeweils ein ionisches und ein amphoteres Polymer enthalten.

Die Polymere sind in den erfindungsgemäß verwendeten Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Die farberhaltende Wirkung des erfindungsgemäßen Wirkstoffkombination kann auch durch eine 2-Pyrrolidinon-5-carbonsäure und deren Derivate gesteigert werden. Ein weiterer Gegenstand der Erfindung ist daher die Verwendung der farberhaltenden Wirkstoffkombination in Kombination mit Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Weiterhin können in den erfindungsgemäß verwendeten Zubereitungen Proteinhydrolysate enthalten sein. Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Index) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda) oder Crotein® (Croda) vertrieben.

Die Proteinhydrolysate oder deren Derivate sind in den erfindungsgemäß verwendeten Mitteln bevorzugt in Mengen von 0,1 bis 10 Gew.-%, bezogen auf das gesamte Mittel, enthalten. Mengen von 0,1 bis 5 Gew.-% sind besonders bevorzugt.

Ebenfalls als vorteilhaft hat sich die Kombination der farberhaltenden Wirkstoffkombination mit Tensiden erwiesen. In einer weiteren bevorzugten Ausführungsform enthalten die erfindungsgemäß verwendeten Mittel Tenside. Unter dem Begriff Tenside werden oberflächenaktive Substanzen verstanden, welche eine anionische oder kationische Ladung im Molekül tragen. Ebenfalls kann im Molekül sowohl eine anionische als auch kationische Ladung vorhanden sein. Diese zwitterionischen oder amphoteren oberflächenaktiven Substanzen können erfindungsgemäß ebenfalls eingesetzt werden. Weiterhin können die oberflächenaktiven Substanzen auch nicht-ionisch sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (VIII), 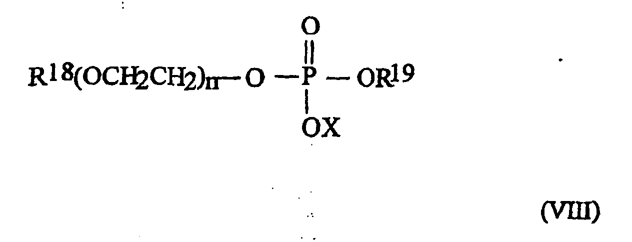 in der R¹⁸ bevorzugt für einen aliphatischen Kohlenwasserstoffrest mit 8 bis 30 Kohlenstoffatomen, R¹⁹ für Wasserstoff, einen Rest (CH₂CH₂O)ₙR¹⁸ oder X, n für Zahlen von 1 bis 10 und X für Wasserstoff, ein Alkali- oder Erdalkalimetall oder NR²⁰R²¹R²²R²³, mit R²⁰ bis R²³ unabhängig voneinander stehend für Wasserstoff oder einen C₁ bis C₄ - Kohlenwasserstoffrest, steht,
- sulfatierte Fettsäurealkylenglykolester der Formel (IX)

   R²⁴CO(AlkO)ₙSO₃M (IX)

   in der R²⁴CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (X) 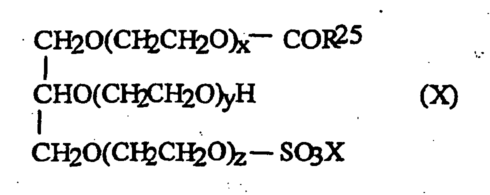 in der R²⁵CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (X) eingesetzt, in der R²⁵CO für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen. Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂-C₁₈ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (XI)

   R¹⁶CO-(OCH₂CHR²⁷)_{w}OR²⁸ (XI)

   in der R²⁶CO für einen linearen oder verzweigten, gesättigten und/oder ungesättigten Acylrest mit 6 bis 22 Kohlenstoffatomen, R²⁷ für Wasserstoff oder Methyl, R²⁸ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Diese Tenside werden in Mengen von 0,1 - 45 Gew.%, bevorzugt 1 - 30 Gew.% und ganz besonders bevorzugt von 1 - 15 Gew.%, bezogen auf das gesamte erfindungsgemäß verwendete Mittel, eingesetzt.

Erfindungsgemäß einsetzbar sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Anionische, nichtionische, zwitterionische und/oder amphotere Tenside sowie deren Mischungen können erfindungsgemäß bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zukkerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C - Atomen und deren Na-, K-, Ammonium-, Ca-, Mg- und Zn - Salze.

Die erfindungsgemäß verwendeten Mittel enthalten die Emulgatoren bevorzugt in Mengen von 0,1 - 25 Gew.-%, insbesondere 0,5 *-* 15 Gew.-%, bezogen auf das gesamte Mittel.

Bevorzugt können die Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Ebenfalls als vorteilhaft hat sich die Kombination der farberhaltenden Wirkstoffkombination mit Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur **Vitamin B-Gruppe** oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

**Vitamin C** (Ascorbinsäure). Vitamin C wird in den erfindungsgemäß verwendeten Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

**Vitamin E** (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Mitteln bevorzugt in Mengen von 0,05-1 Gew.-%, bezogen auf das gesamte Mittel, enthalten.

**Vitamin F.** Unter dem Begriff "Vitamin F" werden üblicherweise essentielle Fettsäuren, insbesondere Linolsäure, Linolensäure und Arachidonsäure, verstanden.

**Vitamin H.** Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäß verwendeten Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Mittel Vitamine, Provitamine und Vitaminvorstufen aus den Gruppen A, B, E und H.

Panthenol und seine Derivate sowie Nicotinsäureamid und Biotin sind besonders bevorzugt.

Schließlich läßt sich die Wirkung der farberhaltenden Wirkstoffkombination auch durch den kombinierten Einsatz mit Pflanzenextrakten steigern.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäß verwendeten Mitteln Mischungen aus mehreren, insbesondere aus zwei, verschiedenen Pflanzenextrakten einzusetzen.

Die erfindungsgemäße farberhaltende Wirkstoffkombination kann prinzipiell direkt dem Färbemittel zugegeben werden. Das Aufbringen der farberhaltenden Wirkstoffkombination auf die gefärbte keratinische Faser kann aber auch in einem getrennten Schritt, entweder vor oder im Anschluß an den eigentlichen Färbevorgang erfolgen. Auch getrennte Behandlungen, gegebenenfalls auch Tage oder Wochen vor oder nach dem Färbevorgang werden von der erfindungsgemäßen Lehre umfaßt. Bevorzugt erfolgt jedoch die Anwendung der erfindungsgemäßen Wirkstoffkombination vor der Färbung und insbesondere im Färbemittel.

Der Begriff Färbevorgang umfaßt dabei alle dem Fachmann bekannten Verfahren, bei denen auf das, gegebenenfalls angefeuchtete, Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Hinsichtlich der Art, gemäß der die erfindungsgemäße farberhaltende Wirkstoffkombination auf die keratinische Faser, insbesondere das menschliche Haar, aufgebracht wird, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Der pH-Wert dieser Zubereitungen kann prinzipiell bei Werten von 2 - 11 liegen. Er liegt bevorzugt zwischen 5 und 11, wobei Werte von 6 bis 10 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Monoethanolamin, Triethanolamin sowie N,N,N',N'-Tetrakis-(2-hydroxypropyl)-ethylendiamin.

Auf dem Haar verbleibende Zubereitungen haben sich als besonders wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d. h. in der Regel mehr als 12 Stunden, auf dem Haar.

Gemäß einer bevorzugten Ausführungsform werden diese Zubereitungen als Haarkur oder Haar-Conditioner formuliert. Die erfindungsgemäß verwendeten Zubereitungen gemäß dieser Ausführungsform können nach Ablauf dieser Einwirkzeit mit Wasser oder einem zumindest überwiegend wasserhaltigen Mittel ausgespült werden; bevorzugt werden sie jedoch, wie oben ausgeführt, auf dem Haar belassen. Dabei kann es bevorzugt sein, die Zubereitung vor der Anwendung eines reinigenden Mittels, eines Wellmittels oder anderen Haarbehandlungsmitteln auf das Haar aufzubringen. In diesem Falle dient die Zubereitung als Farbschutz für die nachfolgenden Anwendungen.

Gemäß weiteren Ausführungsformen kann es sich bei den erfindungsgemäß verwendeten Mitteln aber beispielsweise auch um reinigende Mittel wie Shampoos, pflegende Mittel wie Spülungen, festigende Mittel wie Haarfestiger, Schaumfestiger, Styling Gels und Fönwellen, dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

Neben der erfindungsgemäß zwingend erforderlichen farberhaltenden Wirkstoffkombination und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionische Polymere wie beispielsweise Vinylpyrrolidon/Vinylacrylat-Copoly mere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Fettalkohole, insbesondere lineare und/oder gesättigte Fettalkohole mit 8 bis 30 C-Atomen, und Monoester der Fettsäuren mit Alkoholen mit 6 bis 24 C-Atomen,
- faserstrukturverbessemde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- weitere Substanzen zur Einstellung des pH-Wertes, wie beispielsweise α- und β-Hydroxycarbonsäuren
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Prapylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die oben genannte Monographie von Kh. Schrader verwiesen.

Wie bereits oben erwähnt, kann es im Rahmen der erfindungsgemäßen Lehre bevorzugt sein, die farberhaltende Wirkstoffkombination direkt in die Färbe- oder Tönungsmittel einzuarbeiten.

Die Zusammensetzung des Färbe- oder Tönungsmittels unterliegt keinen prinzipiellen Einschränkungen.
Als Farbstoff(vorprodukt)e können
- Oxidationsfarbstoffvorprodukte vom Entwickler- und Kuppler-Typ,
- natürliche und synthetische direktziehende Farbstoffe und
- Vorstufen naturanaloger Farbstoffe, wie Indol- und Indolin-Derivate,
sowie Mischungen von Vertretern einer oder mehrerer dieser Gruppen eingesetzt werden.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivafe verwendet. Beispiele für solche Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide.

Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

### Beispiele

### Alle Mengenangaben sind, soweit nicht anders vermerkt, Gewichtsteile.

### 1. Wirkungsnachweis

Es wurden die Haarfärbecremebasen der in der folgenden Tabelle aufgeführten Zusammensetzungen hergestellt. Die Bestandteile wurden der Reihe nach miteinander vermischt. Die oxidative Entwicklung der Färbung wurde in einer Anmischung im Verhältnis 5:4 mit 5%iger Wasserstoffperoxid-Dispersion durchgeführt. Die Einwirkzeit auf dem Haar der Fa. Kerling, Typ "Naturweiß", betrug 30 Minuten bei 25°C. Nach der Beendigung des Färbeprozesses wurde das Haar gespült und mit einer wäßrigen Lösung, bestehend aus 1,0 Gew.% Texapon® NSO, pH - Wert 6 - 7, gewaschen und anschließend getrocknet. Jede Haarsträhne wurde an acht Stellen mit Hilfe eines Farbmeßsystemes der Firma Datacolor vermessen. Dabei wurde die zu vermessende Probe in einer Einspannvorrichtung am Spektralphotometer fixiert, die Remissionswerte über den Bereich des sichtbaren Lichtes von 390 - 700 nm im Abstand von 10 nm gemessen und über einen Rechner verarbeitet. Das Rechnerprogramm ermittelte die Normfarbwerte nach dem CIE-System entsprechend DIN 5033. Als Standard bezüglich Farbintensität und Waschechtheit diente die jeweils mit "V" gekennzeichnete Zusammensetzung. Dazu wurde die Farbintensität der mit diesen Zusammensetzungen gefärbten, gewaschenen Strähnen farbmetrisch bestimmt und zu 100% gesetzt. Die dazu relativen Farbintensitäten der mit den übrigen Zusammensetzungen gefärbten, gewaschenen Strähnen ist der Tabelle zu entnehmen. Die Restfarbstärke der weitere 6 mal gewaschenen Haarsträhnen wurde relativ zur 1 mal gewaschenen Haarsträhne bestimmt. Diese Werte sind ebenfalls der Tabelle zu entnehmen.

| Zusammensetzung / Wirkung | Braun 1 | Braun V1 | Kupfer 2 | Kupfer V2 | Granat 3 | Granat V3 |
|---|---|---|---|---|---|---|
| Lamesoft® PO 65 (Cognis) Coco Glucosid (and) Glyceryl Oleate | 3,0 | --- | 3,0 | --- | 3,0 | --- |
| Texapon® NSO (Cognis, ca. 28%) Sodium Laureth Sulfate | 12,0 | 15,0 | 12,0 | 15,0 | 12,0 | 15,0 |
| Dehyton® K (Cognis, ca 30%) | 12,5 | 12,5 | 12,5 | 12,5 | 12,5 | 12,5 |
| Kokoslorol C12-18 (Cognis) | 2,0 | 2,0 | 2,0 | 2,0 | 2,0 | 2,0 |
| Hydrenol® D (Cognis) | 8,5 | 8,5 | 8,5 | 8,5 | 8,5 | 8,5 |
| Eumulgin® B2 (Cognis) | 0,75 | 0,75 | 0,75 | 0,75 | 0,75 | 0,75 |
| Ascorbinsäure | 0,4 | 0,4 | 0,4 | 0,4 | 0,4 | 0,4 |
| Natriumsulfit | 0,4 | 0,4 | 0,4 | 0,4 | 0,4 | 0,4 |
| Arginin | --- | --- | --- | --- | 1,0 | 1,0 |
| Natriumsilicat | --- | --- | 0,1 | 0,1 | --- | --- |
| Gluadin® W40 (Cognis, ca. 40%) | 2,0 | 2,0 | --- | --- | --- | --- |
| Ammoniak | ad pH 8-9 | ad pH 8-9 | ad pH 8-9 | ad pH 8-9 | ad pH 8-9 | ad pH 8-9 |
| p-Toluylendiaminsulfat | 0,6 | 0,6 | 0,2 | 0,2 | 0,13 | 0,13 |
| 2-Methylresorcin | 0,3 | 0,3 | 0,08 | 0,08 | 0,7 | 0,7 |
| 4-Chlorresorcin | 0,06 | 0,06 | 0,3 | 0,3 | --- | --- |
| Tetraaminopyrimidinsulfat | --- | --- | 0,36 | 0,36 | 1,2 | 1,2 |
| 3-Methyl-4-aminophenol | --- | --- | 0,44 | 0,44 | --- | --- |
| 2,7-Dihydroxynaphthalin | --- | --- | 0,3 | 0,3 | --- | --- |
| 2-Amino-6-chlor-4-nitrophenol | --- | --- | 0,1 | 0,1 | --- | --- |
| 1-(2'-Hydroxyethyl)amino-4-methyl-2-nitrobenzol | --- | --- | --- | --- | 0,05 | 0,05 |
| Wasser | ad 100 | | | | | |
| Farbintensität in % | 115 | 100 | 111 | 100 | 107 | 100 |
| Restfarbstärke in % | 89 | 83 | 92 | 62 | 99 | 93 |

### 2. Anwendungsbeispiele

### 2.1. Haarspülung

| | |
|---|---|
| Eumulgin® B2¹ | 0,3 |
| Cetyl/Stearylalkohol | 3,3 |
| Isopropylmyristat | 0,5 |
| Lamesoft® PO 65 | 0,5 |
| Dehyquart®A-CA² | 2,0 |
| Salcare®SC 96³ | 1,0 |
| Citronensäure | 0,4 |
| Gluadin® WQ⁴ | 2,0 |
| Phenonip®⁵ | 0,8 |
| Wasser | ad 100 |

| | |
|---|---|
| ¹ Cetylstearylalkohol + 20 EO (INCI-Bezeichnung: Ceteareth-20) (COGNIS) ² Trimethylhexadecylammoniumchlorid ca. 25% Aktivsubstanz (INCI-Bezeichnung: Cetrimonium Chloride) (COGNIS) ³ N,N,N-Trimethyl-2[(methyl-1-oxo-2-propenyl)oxy]-Ethanaminiumchlorid-Homopolymer (50 % Aktivsubstanz; INCI-Bezeichnung: Polyquaternium-37 (and) Propylenglycol Dicaprilate Dicaprate (and) PPG-1 Trideceth-6) (ALLIED COLLOIDS) ⁴ Kationisiertes Weizenproteinhydrolysat ca. 31% Aktivsubstanz (INCI-Bezeichnung: Laurdimonium Hydroxypropyl Hydrolyzed Wheat Protein) (COGNIS) ⁵ Hydroxybenzoesäuremethylester-Hydroxybenzoesäureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben, Butylparaben) (NIPA) | |

### 2.2 Haarspülung

| | |
|---|---|
| Eumulgin® B2 | 0,3 |
| Cetyl/Stearylalkohol | 3,3 |
| Isopropylmyristat | 0,5 |
| Paraffinöl perliquidum 15 cSt. DAB 9 | 0,3 |
| Dehyquart®L 80⁶ | 0,4 |
| Lamesoft® PO 65 | 1,5 |
| Cosmedia Guar® C 261 ⁷ | 1,5 |
| Promois® Milk-CAQ⁸ | 3,0 |
| Citronensäure | 0,4 |
| Phenonip® | 0,8 |
| Wasser | ad 100 |

| | |
|---|---|
| ⁶ Bis(cocoylethyl)-hydroxyethyl-methyl-ammonium-methosulfat (ca. 76 % Aktivsubstanz in Propylenglykol; INCI-Bezeichnung: Dicocoylethyl Hydroxyethylmonium Methosulfat, Propylene Glycol) (COGNIS) ⁷ Guarhydroxypropyltrimethylammonium Chlorid; INCI-Bezeichnung: Guar Hydroxypropyl Trimonium Chloride (COGNIS) ⁸ INCI-Bezeichnung: Cocodimonium Hydroxypropyl Hydrolyzed Casein (SEIWA KASEI) | |

### 2.3. Haarkur

| | |
|---|---|
| Dehyquart® F75⁹ | 4,0 |
| Cetyl/Stearylalkohol | 4,0 |
| Paraffinöl perliquidum 15 cSt DAB 9 | 1,5 |
| Dehyquart®A-CA | 4,0 |
| Lamesoft® PO 65 | 1,0 |
| Salcare®SC 96 | 1,5 |
| Amisafe-LMA-60®¹⁰ | 1,0 |
| Gluadin®W 20¹¹ | 3,0 |
| Citronensäure | 0,15 |
| Phenonip® | 0,8 |
| Wasser | ad 100 |

| | |
|---|---|
| ⁹ Fettalkohole-Methyltriethanolammoniummethylsulfatdialkylester-Gemisch (INCI-Bezeichnung: Distearoylethyl Hydroxyethylmonium Methosulfate, Cetearyl Alcohol) (COGNIS) ¹⁰ INCI-Bezeichnung Hydroxypropyl Arginine Lauryl/Myristyl Ether HCl (Ajinomoto) ¹¹ Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (COGNIS) | |

### 2.4. Haarkur

| | |
|---|---|
| Dehyquart® L80 | 2,0 |
| Cetyl/Stearylalkohol | 6,0 |
| Paraffinöl perliquidum 15 cSt DAB 9 | 2,0 |
| Rewoquat®W 75¹² | 2,0 |
| Cosmedia Guar® C261 | 0,5 |
| Lamesoft® PO 65 | 0,5 |
| Sepigel®305¹³ | 3,5 |
| Honeyquat® 50¹⁴ | 1,0 |
| Gluadin® WQ | 2,5 |
| Gluadin®W 20 | 3,0 |
| Citronensäure | 0,15 |
| Phenonip® | 0,8 |
| Wasser | ad 100 |

| | |
|---|---|
| ¹² 1-Methyl-2-nortalgalkyl-3-talgfettsäureamidoethylimidazolinium-methosulfat (ca. 75 % Aktivsubstanz in Propylenglykol; INCI-Bezeichnung: Quaternium-27, Propylene Glycol) (WITCO) ¹³ Copolymer aus Acrylamid und 2-Acrylamido-2-methylpropansulfonsäure (INCI-Bezeichnung: Polyacrylamide (and) C₁₃-C₁₄ Isoparaffin (and) Laureth-7) (SEPPIC) ¹⁴ INCI - Bezeichnung: Hydroxypropyltrimonium Honey (BROOKS) | |

### 2.5. Haarkur

| | |
|---|---|
| Dehyquart® F75 | 0,3 |
| Salcare®SC 96 | 5,0 |
| Gluadin® WQ | 1,5 |
| Lamesoft® PO 65 | 0,5 |
| Dow Corning®200 Fluid, 5 cSt.¹⁵ | 1,5 |
| Gafquat®755N¹⁶ | 1,5 |
| Biodocarb® ¹⁷ | 0,02 |
| Parfümöl | 0,25 |
| Wasser | ad 100 |

| | |
|---|---|
| ¹⁵ Polydimethylsiloxan (INCI-Bezeichnung: Dimethicone) (DOW CORNING) ¹⁶ Dimethylaminoethylmethacrylat-Vinylpyrrolidon-Copolymer, mit Diethylsulfat quaterniert (19 % Aktivsubstanz in Wasser; INCI-Bezeichnung: Polyquaternium-11) (GAF) ¹⁷ 3-Iod-2-propinyl-n-butylcarbamat (INCI-Bezeichnung: Iodopropynyl Butylcarbamate) (MILKER & GRÜNING) | |

### 2.6. Haarkur

| | |
|---|---|
| Sepigel®305 | 5,0 |
| Dow Corning®Q2-5220¹⁸ | 1,5 |
| Promois® Milk Q¹⁹ | 3,0 |
| Lamesoft® PO 65 | 0,5 |
| Polymer P1 entsprechend DE 3929173 | 0,6 |
| Genamin®DSAC²⁰ | 0,3 |
| Phenonip® | 0,8 |
| Parfümöl | 0,25 |
| Wasser | ad 100 |

| | |
|---|---|
| ¹⁸ Silicon-Glykol-Copolymer (INCI-Bezeichnung: Dimethicone Copolyol) (DOW CORNING) ¹⁹ INCI-Bezeichnung Hydroxypropyltrimonium Hydrolyzed Casein ca. 30% Aktivsubstanz (SEIWA KASEI) ²⁰ Dimethyldistearylammoniumchlorid (INCI-Bezeichnung: Distearyldimonium Chloride) (CLARIANT) | |

### 2.7. Shampoo

| | |
|---|---|
| Texapon® NSO²¹ | 40,0 |
| Dehyton® G²² | 6,0 |
| Polymer JR 400®²³ | 0,5 |
| Cetiol® HE²⁴ | 0,5 |
| Ajidew® NL 50²⁵ | 1,0 |
| Lamesoft® PO 65 | 3,0 |
| Gluadin® WQT²⁶ | 2,5 |
| Gluadin® W 20 | 0,5 |
| Panthenol (50%) | 0,3 |
| Vitamin E | 0,1 |
| Vitamin H | 0,1 |
| Citronensäure | 0,5 |
| Natriumbenzoat | 0,5 |
| Parfüm | 0,4 |
| NaCl | 0,5 |
| Wasser | ad 100 |

| | |
|---|---|
| ²¹ Natriumlaurylethersulfat ca. 28% Aktivsubstanz (INCI - Bezeichnung: Sodium Laureth Sulfate) (COGNIS) ²² (INCI - Bezeichnung: Sodium Cocoamphoacetate, ca. 30% Aktivsubstanz in Wasser) (COGNIS) ²³ quaternierte Hydroxyethylcellulose (INCI - Bezeichnung: Polyquaternium-10) (UNION CARBIDE) ²⁴ Polyol-Fettsäure-Ester (INCI - Bezeichnung: PEG-7 Glyceryl Cocoate) (COGNIS) ²⁵ Natrium-Salz der 2-Pyrrolidinon-5-carbonsäure (50% Aktivsubstanz: INCI-Bezeichnung: Sodium PCA) (AJINOMOTO) ²⁶ INCI-Bezeichnung: Hydroxypropyltrimonium Hydrolyzed Wheat Protein (COGNIS) | |

### 2.8. Shampoo

| | |
|---|---|
| Texapon® NSO | 43,0 |
| Dehyton® K²⁷ | 10,0 |
| Plantacare® 1200 UP²⁸ | 4,0 |
| Lamesoft® PO 65 | 2,5 |
| Euperlan®PK 3000²⁹ | 1,6 |
| Arquad®316³⁰ | 0,8 |
| Polymer JR® 400 | 0,3 |
| Gluadin® WQ | 4,0 |
| Glucamate®DOE 120³¹ | 0,5 |
| Natriumchlorid | 0,2 |
| Wasser | ad 100 |

| | |
|---|---|
| ²⁷ INCI - Bezeichnung: Cocamidopropyl Betaine ca. 30% Aktivsubstanz (COGNIS) ²⁸ C 12 - C 16 Fettalkoholglycosid ca. 50% Aktivsubstanz (INCI - Bezeichnung: Lauryl Glucoside) (COGNIS) ²⁹ Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (COGNIS) ³⁰ Tri-C₁₆-alkylmethylammoniumchlorid (AKZO) ³¹ ethoxyliertes Methylglucosid-dioleat (CTFA-Bezeichnung: PEG-120 Methyl Glucose Dioleate) (AMERCHOL) | |

### 2.9. Shampoo

| | |
|---|---|
| Texapon®N 70³² | 21,0 |
| Plantacare® 1200 UP | 8,0 |
| Lamesoft® PO 65 | 3,0 |
| Gluadin® WQ | 1,5 |
| Cutina® EGMS³³ | 0,6 |
| Honeyquat® 50³⁴ | 2,0 |
| Ajidew® NL 50 | 2,8 |
| Antil® 141³⁵ | 1,3 |
| Natriumchlorid | 0,2 |
| Magnesiumhydroxid | ad pH 4,5 |
| Wasser | ad 100 |

| | |
|---|---|
| ³² Natriumlaurylethersulfat mit 2 Mol EO ca. 70% Aktivsubstanz (INCI - Bezeichnung: Sodium Laureth Sulfate) (COGNIS) ³³ Ethylenglykolmonostearat (ca. 25-35% Monoester, 60-70% Diester; INCI:Bezeichnung: Glycol Stearate) (COGNIS) ³⁴ INCI-Bezeichnung: Hydroxypropyltrimonium Honey (ca. 50% Aktivsubstanz) (BROOKS) ³⁵ Polyoxyethylen-propylenglykoldioleat (40 % Aktivsubstanz; INCI - Bezeichnung: Propylene Glycol (and) PEG-55 Propylene Glycol Oleate) (GOLDSCHMIDT) | |

### 2.10. Shampoo

| | |
|---|---|
| Texapon® K 14 S³⁶ | 50,0 |
| Dehyton® K | 10,0 |
| Plantacare® 818 UP³⁷ | 4,5 |
| Lamesoft® PO 65 | 2,0 |
| Polymer P1, entsprechend DE 39 29 973 | 0,6 |
| Cutina® AGS³⁸ | 2,0 |
| D-Panthenol | 0,5 |
| Glucose | 1,0 |
| Salicylsäure | 0,4 |
| Natriumchlorid | 0,5 |
| Gluadin® WQ | 2,0 |
| Wasser | ad 100 |

| | |
|---|---|
| ³⁶ Natriumlaurylmyristylethersulfat ca 28% Aktivsubstanz (INCI - Bezeichnung: Sodium Myreth Sulfate) (COGNIS) ³⁷ C 8 - C 16 Fettalkoholglycosid ca. 50% Aktivsubstanz (INCI - Bezeichnung: Coco Glucoside) (COGNIS) ³⁸ Ethylenglykolstearat (ca. 5-15% Monoester, 85-95% Diester; INCI - Bezeichnung: Glycol Distearate) (COGNIS) | |

### 2.11. Haarkur

| | |
|---|---|
| Celquat® L 200³⁹ | 0,6 |
| Luviskol® K30⁴⁰ | 0,2 |
| D-Panthenol | 0,5 |
| Polymer P1, entsprechend DE 39 29 973 | 0,6 |
| Dehyquart® A-CA⁴¹ | 1,0 |
| Lamesoft® PO 65 | 0,5 |
| Gluadin® W 40⁴² | 1,0 |
| Natrosol® 250 HR⁴³ | 1,1 |
| Gluadin® WQ | 2,0 |
| Wasser | ad 100 |

| | |
|---|---|
| ³⁹ quaterniertes Cellulose-Derivat (95 % Aktivsubstanz; CTFA-Bezeichnung: Polyquaternium-4) (DELFT NATIONAL) ⁴⁰ Polyvinylpyrrolidon (95 % Aktivsubstanz; CTFA-Bezeichnung: PVP) (BASF) ⁴¹ Cetyltrimethylammoniumchlorid (INCI - Bezeichnung: Cetrimonium Chloride) (COGNIS) ⁴² Partialhydrolysat aus Weizen ca. 40% Aktivsubstanz (INCI - Bezeichnung: Hydrolyzed Wheat Gluten Hydrolyzed Wheat Protein) (COGNIS) ydroxyethylcellulose (AQUALON) | |

### 2.12. Färbecreme

| | |
|---|---|
| C₁₂₋₁₈-Fettalkohol | 1,2 |
| Lanette® O⁴⁴ | 4,0 |
| Eumulgin® B 2 | 0,8 |
| Cutina® KD 16⁴⁵ | 2,0 |
| Lamesoft® PO 65 | 4,0 |
| Natriumsulfit | 0,5 |
| L(+)-Ascorbinsäure | 0,5 |
| Ammoniumsulfat | 0,5 |
| 1,2-Propylenglykol | 1,2 |
| Polymer JR®400 | 0,3 |
| p-Aminophenol | 0,35 |
| p-Toluylendiamin | 0,85 |
| 2-Methylresorcin | 0,14 |
| 6-Methyl-m-aminophenol | 0,42 |
| Cetiol® OE⁴⁶ | 0,5 |
| Honeyquat® 50 | 1,0 |
| Ajidew® NL 50 | 1,2 |
| Gluadin® WQ | 1,0 |
| Ammoniak | 1,5 |
| Wasser | ad 100 |

| | |
|---|---|
| ⁴⁴ Cetylstearylalkohol (INCI - Bezeichnung: Cetearyl Alcohol) (COGNIS) ⁴⁵ Selbstemulgierendes Gemisch aus Mono- / Diglyceriden höherer gesättigter Fettsäuren mit Kaliumstearat (INCI - Bezeichnung: Glyceryl Stearate SE) (COGNIS) ⁴⁶ Di-n-octylether (INCI - Bezeichnung: Dicaprylyl Ether) (COGNIS) | |

### 2.13. Entwicklerdispersion für Färbecreme 2.12.

| | |
|---|---|
| Texapon® NSO | 2,1 |
| Wasserstoffperoxid (50%ig) | 12,0 |
| Turpinal® SL⁴⁷ | 1,7 |
| Latekoll® D⁴⁸ | 12,0 |
| Lamesoft® PO 65 | 2,0 |
| Gluadin® WQ | 0,3 |
| Salcare® SC 96 | 1,0 |
| Wasser | ad 100 |

| | |
|---|---|
| ⁴⁷ 1-Hydroxyethan-1,1-diphosphonsäure (60 % Aktivsubstanz; INCI - Bezeichnung: Etidronic Acid) (COGNIS) ⁴⁸ Acrylester-Methacrylsäure-Copolymer (25 % Aktivsubstanz) (BASF) | |

Die Färbecreme hatte einen pH-Wert von 10,0. Sie bewirkte eine intensive rote Tönung des Haares.

### 2.14. Tönungsshampoo

| | |
|---|---|
| Texapon® N 70 | 14,0 |
| Dehyton® K | 10,0 |
| Akypo® RLM 45 NV⁴⁹ | 14,7 |
| Plantacare® 1200 UP | 4,0 |
| Lamesoft® PO 65 | 3,0 |
| Polymer P1, entsprechend DE 39 29 973 | 0,3 |
| Cremophor® RH 44⁵⁰ | 0,8 |
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| Konservierung | 0,25 |
| Parfümöl | q.s. |
| Eutanol® G⁵¹ | 0,3 |
| Gluadin® WQ | 1,0 |
| Honeyquat® 50 | 1,0 |
| Salcare® SC 96 | 0,5 |
| Wasser | ad 100 |

| | |
|---|---|
| ⁴⁹ Laurylalkohol+4,5 Ethylenoxid-essigsäure-Natriumsalz (20,4 % Aktivsubstanz) (CHEM-Y) ⁵⁰ Rizinus-Öl, hydriert + 45 Ethylenoxid (INCI - Bezeichnung: PEG-40 Hydrogenated Castor Oil) (BASF) ⁵¹ 2-Octyldodecanol (Guerbet-Alkohol) (INCI - Bezeichnung: Octyldodecanol) (COGNIS) | |

Beim Waschen der Haare mit diesem Tönungs-Shampoo erhalten diese einen glänzenden, hellblonden Farbton.

### 2.15. Cremedauerwelle

### Wellcreme

| | |
|---|---|
| Plantacare® 810 UP⁵² | 5,0 |
| Thioglykolsäure | 8,0 |
| Turpinal® SL | 0,5 |
| Ammoniak (25%ig) | 7,3 |
| Ammoniumcarbonat | 3,0 |
| Cetyl/Stearyl-Alkohol | 5,0 |
| Lamesoft® PO 65 | 0,5 |
| Guerbet-Alkohol | 4,0 |
| Salcare® SC 96 | 3,0 |
| Gluadin® WQ | 2,0 |
| Parfümöl | q.s. |
| Wasser | ad 100 |

| | |
|---|---|
| ⁵² C₈-C₁₀-Alkylglucosid mit Oligomerisationsgrad 1,6 (ca. 60% Aktivsubstanz) (COGNIS) | |

### Fixierlösung

| | |
|---|---|
| Plantacare® 810 UP | 5,0 |
| gehärtetes Rizinusöl | 2,0 |
| Lamesoft® PO 65 | 1,0 |
| Kaliumbromat | 3,5 |
| Nitrilotriessigsäure | 0,3 |
| Zitronensäure | 0,2 |
| Merquat® 550⁵³ | 0,5 |
| Hydagen® HCMF⁵⁴ | 0,5 |
| Gluadin® WQ | 0,5 |
| Parfümöl | q.s. |
| Wasser | ad 100 |

| | |
|---|---|
| ⁵³ Dimethyldiallylammoniumchlorid-Acrylamid-Copolymer (8 % Aktivsubstanz; INCI - Bezeichnung: Polyquarternium 7) (MOBIL OIL) ⁵⁴ Chitosan Pulver (INCI - Bezeichnung: Chitosan) (COGNIS) | |

## Patentansprüche

1. Verwendung einer Wirkstoffkombination aus:
a) mindestens einem Zuckertensid (A), ausgewählt aus der Gruppe, die gebildet wird
von Alkyl- und Alkenyloligoglykosiden (A1) und Fettsäure-N-alkylpolyhydroxyalkylamiden (A2), und
b) mindestens einem Fettsäurepartialglycerid (B)
zur Verbesserung der Waschechtheit von Färbungen von Fasern, insbesondere von keratinischen Fasern, sowie zur Steigerung der Farbintensität.

2. Verwendung nach Anspruch 1, **dadurch gekennzeichnet, daß** das die Wirkstoffkombination enthaltende Mittel zusätzlich ein Polymer enthält.

3. Verwendung nach einem der Ansprüche 1 bis 2, **dadurch gekennzeichnet, daß** das Polymer ein kationisches oder amphoteres Polymer ist.

4. Verwendung nach Anspruch 3, **dadurch gekennzeichnet, daß** das kationische Polymer ausgewählt ist aus Homopolymeren der allgemeinen Formel (I), 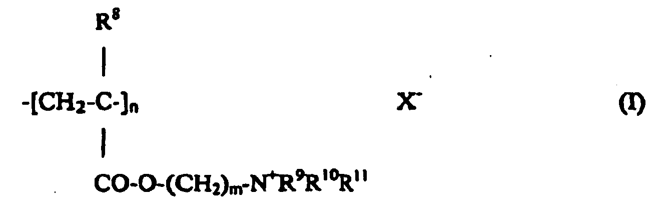 in der R⁸ = -H oder -CH₃ ist, R⁹, R¹⁰ und R¹¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, und Copolymeren, bestehend im wesentlichen aus den in Formel (I) aufgeführten Monomereinheiten sowie nichtionogenen Monomereinheiten.

5. Verwendung nach Anspruch 3, **dadurch gekennzeichnet, daß** die kationischen Polymere ausgewählt sind aus der Gruppe, die von
- kationisierten Cellulose-Derivaten,
- kationisierten Guar-Derivaten,
- kationisiertem Honig und
- kationischen Alkylpolyglycosiden gebildet wird.

6. Verwendung nach Anspruch 2, **dadurch gekennzeichnet, daß** das Polymer ein anionisches Polymer ist.

7. Verwendung nach Anspruch 6, **dadurch gekennzeichnet, daß** das anionische Polymer Carboxylat- und/oder Sulfonatgruppen aufweist.

8. Verwendung nach Anspruch 7, **dadurch gekennzeichnet, daß** das anionische Polymer als Monomer 2-Acrylamido-2-methylpropansulfonsäure enthält, wobei die Sulfonsäuregruppe ganz oder teilweise als Natrium-, Kalium-, Ammonium-, Mono- oder Triethanolammonium-Salz vorliegen kann.

9. Verwendung nach einem der Ansprüche 6 bis 8, **dadurch gekennzeichnet, daß** das anionische Polymer ein Copolymer aus mindestens einem anionischen Monomer und mindestens einem nichtionogenen Monomer ist.

10. Verwendung nach Anspruch 9, **dadurch gekennzeichnet, daß** das anionische Polymer ein Copolymer ist, das besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt.

11. Verwendung nach einem der Ansprüche 1 bis 10, **dadurch gekennzeichnet, daß** das die Wirkstoffkombination enthaltende Mittel weiterhin ein Proteinhydrolysat oder ein Derivat eines Proteinhydrolysats enthält.

12. Verwendung nach Anspruch 11, **dadurch gekennzeichnet, daß** das Proteinhydrolysat oder Derivat des Proteinhydrolysats pflanzlichen Ursprungs ist.

13. Verwendung nach einem der Ansprüche 1 bis 12, **dadurch gekennzeichnet, daß** das die Wirkstoffkombination enthaltende Mittel weiterhin ein Tensid enthält.

14. Verwendung nach Anpruch 13, **dadurch gekennzeichnet, daß** das Tensid ausgewählt ist aus der Gruppe der anionischen, zwitterionischen, amphoteren oder nichtionischen Tenside.

15. Verwendung nach einem der Ansprüche 1 bis 14, **dadurch gekennzeichnet, daß** das die Wirkstoffkombination enthaltende Mittel weiterhin ein Vitamin, ein Provitamin, eine Vitaminvorstufe oder ein Derivat davon enthält.

16. Verwendung nach einem der Ansprüche 1 bis 15, **dadurch gekennzeichnet, daß** das die Wirkstoffkombination enthaltende Mittel weiterhin einen Pflanzenextrakt enthält.

## Claims

1. Use of an active ingredient combination of:
a) at least one sugar surfactant (A) chosen from the group consisting of alkyl and alkenyl oligoglycosides (A1) and fatty acid N-alkylpolyhydroxyalkylamides (A2), and
b) at least one fatty acid partial glyceride (B) for improving the washfastness of colorations of fibres, in particular of keratin fibres, and for increasing the colour intensity.

2. Use according to Claim 1, **characterized in that** the composition comprising the active ingredient combination additionally comprises a polymer.

3. Use according to either Claim 1 or 2, **characterized in that** the polymer is a cationic or amphoteric polymer.

4. Use according to Claim 3, **characterized in that** the cationic polymer is chosen from homopolymers of the general formula (I), 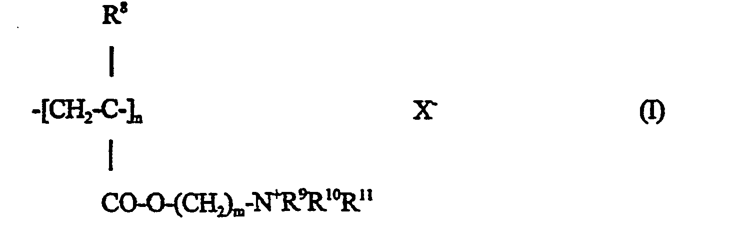 in which R⁸ = -H or -CH₃, R⁹, R¹⁰ and R¹¹, independently of one another, are chosen from C₁₋₄-alkyl, -alkenyl or -hydroxyalkyl groups, m = 1, 2, 3 or 4, n is a natural number and X⁻ is a physiologically compatible organic or inorganic anion, and copolymers consisting essentially of the monomer units listed in formula (I) and also nonionogenic monomer units.

5. Use according to Claim 3, **characterized in that** the cationic polymers are chosen from the group consisting of
- cationized cellulose derivatives,
- cationized guar derivatives,
- cationized honey and
- cationic alkyl polyglycosides.

6. Use according to Claim 2, **characterized in that** the polymer is an anionic polymer.

7. Use according to Claim 6, **characterized in that** the anionic polymer has carboxylate and/or sulphonate groups.

8. Use according to Claim 7, **characterized in that** the anionic polymer comprises, as monomer, 2-acrylamido-2-methylpropanesulphonic acid, where the sulphonic acid group may completely or partially be in the form of the sodium, potassium, ammonium, mono- or triethanolamine salt.

9. Use according to any of Claims 6 to 8, **characterized in that** the anionic polymer is a copolymer of at least one anionic monomer and at least one nonionogenic monomer.

10. Use according to Claim 9, **characterized in that** the anionic polymer is a copolymer which consists of 70 to 55 mol% of acrylamide and 30 to 45 mol% of 2-acrylamido-2-methylpropanesulphonic acid, where the sulphonic acid group is completely or partially present as sodium, potassium, ammonium, mono- or triethanolammonium salt.

11. Use according to any of Claims 1 to 10, **characterized in that** the composition comprising the active ingredient combination further comprises a protein hydrolysate or a derivative of a protein hydrolysate.

12. Use according to Claim 11, **characterized in that** the protein hydrolysate or derivative of the protein hydrolysate is of vegetable origin.

13. Use according to any of Claims 1 to 12, **characterized in that** the composition comprising the active ingredient combination further comprises a surfactant.

14. Use according to Claim 13, **characterized in that** the surfactant is chosen from the group of anionic, zwitterionic, amphoteric or nonionic surfactants.

15. Use according to any of Claims 1 to 14, **characterized in that** the composition comprising the active ingredient combination further comprises a vitamin, a provitamin, a vitamin precursor or a derivative thereof.

16. Use according to any of Claims 1 to 15, **characterized in that** the composition comprising the active ingredient combination further comprises a plant extract.

## Revendications

1. Utilisation d'une combinaison de substances actives constituée par :
a) au moins un tensioactif à base de sucre (A), choisi dans le groupe formé par
des alkyl- et alcényloligoglycosides (A1) et des N-alkylpolyhydroxyalkylamides d'acides gras (A2), et
b) au moins un glycéride partiel d'acide gras (B)
pour améliorer la résistance au lavage de la coloration de fibres, en particulier de fibres kératiniques, ainsi que pour renforcer l'intensité de coloration.

2. Utilisation selon la revendication 1, **caractérisée en ce que** le produit contenant la combinaison de substances actives contient en outre un polymère.

3. Utilisation selon l'une des revendications 1 et 2, **caractérisée en ce que** le polymère est un polymère cationique ou amphotère.

4. Utilisation selon la revendication 3, **caractérisée en ce que** le polymère cationique est choisi parmi les homopolymères de formule générale (I), 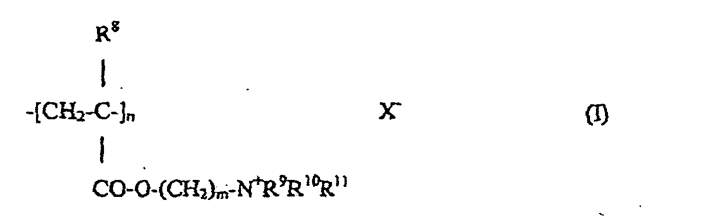 dans laquelle R⁸ = -H ou -CH₃, R⁹, R¹⁰ et R¹¹ représentent indépendamment un groupe alkyle en C₁₋₄, alcényle en C₁₋₄ ou hydroxyalkyle en C₁₋₄, m = 1, 2, 3 ou 4, n est un nombre entier et X⁻ un anion organique ou inorganique physiologiquement acceptable, et parmi les copolymères constitués essentiellement des unités monomères représentées par la formule (I) ainsi que d'unités monomères non ionogènes.

5. Utilisation selon la revendication 3, **caractérisée en ce que** les polymères cationiques sont choisis dans le groupe constitué par
- des dérivés cellulosiques rendus cationiques,
- des dérivés de gomme guar rendus cationiques,
- du miel rendu cationique et
- des alkylpolyglycosides cationiques.

6. Utilisation selon la revendication 2, **caractérisée en ce que** le polymère est un polymère anionique.

7. Utilisation selon la revendication 6, **caractérisée en ce que** le polymère anionique comprend des groupes carboxylate et/ou sulfonate.

8. Utilisation selon la revendication 7, **caractérisée en ce que** le polymère anionique contient, en tant que monomère, l'acide 2-acrylamido-2-méthylpropanesulfonique, le groupe acide sulfonique pouvant être présent partiellement ou totalement sous forme de sel de sodium, de potassium, d'ammonium, de mono- ou triéthanolammonium.

9. Utilisation selon l'une quelconque des revendications 6 à 8, **caractérisée en ce que** le polymère anionique est un copolymère formé d'au moins un monomère anionique et d'au moins un monomère non ionogène.

10. Utilisation selon la revendication 9, **caractérisée en ce que** le polymère anionique est un copolymère formé de 70 à 55 % en moles d'acrylamide et de 30 à 45 % en moles d'acide 2-acrylamido-2-méthylpropanesulfonique, le groupe acide sulfonique pouvant être présent partiellement ou totalement sous forme de sel de sodium, de potassium, d'ammonium, de mono- ou triéthanolammonium.

11. Utilisation selon l'une quelconque des revendications 1 à 10, **caractérisée en ce que** le produit contenant la combinaison de substances actives contient en outre un hydrolysat de protéines ou un dérivé d'hydrolysat de protéines.

12. Utilisation selon la revendication 11, **caractérisée en ce que** l'hydrolysat de protéines ou le dérivé d'hydrolysat de protéines est d'origine végétale.

13. Utilisation selon l'une quelconque des revendications 1 à 12, **caractérisée en ce que** le produit contenant la combinaison de substances actives contient en outre un tensioactif.

14. Utilisation selon la revendication 13, **caractérisée en ce que** le tensioactif est choisi dans le groupe formé par des tensioactifs anioniques, zwitterioniques, amphotères ou non ioniques.

15. Utilisation selon l'une quelconque des revendications 1 à 14, **caractérisée en ce que** le produit contenant la combinaison de substances actives contient en outre une vitamine, une provitamine, un précurseur de vitamine ou un dérivé de ceux-ci.

16. Utilisation selon l'une quelconque des revendications 1 à 15, **caractérisée en ce que** le produit contenant la combinaison de substances actives contient en outre un extrait de plante.
